Europäisches Patentamt

European Patent Office

Office européen des brevets

(11) Veröffentlichungsnummer : **0 110 088 B1**

(12) **EUROPÄISCHE PATENTSCHRIFT**

(45) Veröffentlichungstag der Patentschrift :
16.06.87

(51) Int. Cl.⁴ : **C 07 D301/10, B 01 J 23/96**

(21) Anmeldenummer : **83110331.2**

(22) Anmeldetag : **17.10.83**

(54) **Verfahren zur Regenerierung des Al2O3-Trägermaterials gebrauchter Ag/Al2O3-Trägerkatalysatoren.**

(30) Priorität : **28.10.82 DE 3239886**

(43) Veröffentlichungstag der Anmeldung :
**13.06.84 Patentblatt 84/24**

(45) Bekanntmachung des Hinweises auf die Patenterteilung : **16.06.87 Patentblatt 87/25**

(84) Benannte Vertragsstaaten :
**BE DE FR GB IT NL**

(56) Entgegenhaltungen :
**DE-A- 2 351 661
DE-A- 2 940 480
GB-A- 1 154 528
GB-A- 2 014 133
CHEMICAL ABSTRACTS, Band 89, Nr. 4, Juli 1978, Seite 462, Nr. 31511j, Columbus, Ohio, US**

(73) Patentinhaber : **BASF Aktiengesellschaft
Carl-Bosch-Strasse 38
D-6700 Ludwigshafen (DE)**

(72) Erfinder : **Mross, Wolf Dieter, Dr.
Anselm-Feuerbach-Strasse 21
D-6710 Frankenthal (DE)**
Erfinder : **Schwarzmann, Matthias, Dr.
Carl-Bosch-Strasse 54
D-6703 Limburgerhof (DE)**
Erfinder : **Plueckhan, Juergen, Dr.
Bensheimer Ring 19 b
D-6710 Frankenthal (DE)**
Erfinder : **Dehler, Juergen, Dr.
Im Eichenweg 4
D-6708 Neuhofen (DE)**

## Beschreibung

Die vorliegende Erfindung betrifft die Regenerierung des $Al_2O_3$-Trägermaterials von Ag/$Al_2O_3$-Trägerkatalysatoren, die hauptsächlich für die Herstellung von Ethylenoxid durch Sauerstoffanlagerung an Ethylen Verwendung finden.

Außerdem betrifft die Erfindung die Herstellung von Ethylenoxid unter Verwendung von Ag/$Al_2O_3$-Trägerkatalysatoren, deren Trägermaterial aus dem erfindungsgemäß regenerierten Trägermaterial besteht.

Die Herstellung von Ethylenoxid aus Ethylen und Sauerstoff mittels Silberkontakten in der Gasphase ist in zahlreichen Ausgestaltungen allgemein bekannt. Ebenfalls allgemein bekannt sind die hierfür verwendeten Kontakte, deren aktive Masse im wesentlichen aus Silber besteht und für die in aller Regel Aluminiumoxid — meist $\alpha$-$Al_2O_3$ — als Trägermaterial dient, und zwar in Form von Kugeln mit 3-10 mm Durchmesser oder in sonstiger Form (z. B. Zylinder, Ringe) vergleichbarer Teilchengröße.

Erfahrungsgemäß haben diese Katalysatoren eine Lebensdauer von 2-5 Betriebsjahren, wonach sie in ihrer Wirksamkeit sowohl hinsichtlich des Ethylenumsatzes als auch der Ethylenoxid-Selektivität soweit nachlassen, daß ihre Weiterverwendung nicht mehr wirtschaftlich ist.

Die aktive Masse, besonders wenn sie selektivitätsverbessernde Dotierungen wie Alkalimetallionen enthält, kann zwischenzeitlich durch bestimmte Wasch- und Nachdotierungsverfahren reaktiviert werden, jedoch läßt sich dadurch die Desaktivierung der Katalysatoren nur verzögern, nicht aber grundsätzlich verhindern.

Genügen die Katalysatoren daher nicht mehr den wirtschaftlichen Mindesterfordernissen, müssen sie gegen neue Kontakte ausgetauscht werden. Von den gebrauchten Katalysatoren wurde bisher lediglich das Silber durch Behandlung mit Salpetersäure zurückgewonnen, und das verbleibende $Al_2O_3$-Trägermaterial wurde verworfen (vgl. z. B. DE-A-2 351 661).

Nun bot es sich zwar an, auch das relativ teure Trägermaterial wiederzuverwenden, indem man es mit einer neuen Silberschicht versieht, jedoch stellte sich heraus, daß derartige Katalysatoren deutlich weniger wirksam sind und dementsprechend eine wesentlich geringere Lebensdauer haben als solche mit frischem $Al_2O_3$.

Der Erfindung lag daher die Aufgabe zugrunde, das $Al_2O_3$-Trägermaterial gebrauchter und vom Silber befreiter Ag/$Al_2O_3$-Trägerkatalysatoren wieder für die Herstellung neuer Katalysatoren nutzbar zu machen.

Demgemäß wurde ein Verfahren zur Regenerierung des $Al_2O_3$-Trägermaterials von Ag/$Al_2O_3$-Trägerkatalysatoren, welches nach Ablösung des Silbers von derartigen gebrauchten Katalysatoren mit Salpetersäure anfällt, gefunden, welches dadurch gekennzeichnet ist, daß man das Trägermaterial

a) mit einer wäßrigen Lösung eines wasserlöslichen Salzes oder Hydroxids der Metalle der Gruppen IIA, IIIB oder IVB des Periodensystems oder des Aluminiums, Kupfers, Mangans, Zinks, Cadmiums, Zinns oder Bleis behandelt und das derart behandelte Material anschließend trocknet, und/oder daß man das Material

b) mindestens 10 min lang auf 750-1 500 °C erhitzt.

Die Wirkung dieser Maßnahmen beruht vermutlich darauf, daß saure Zentren, die sich im Trägermaterial bei der Salpetersäurebehandlung bilden, neutralisiert und/oder durch Wassersabspaltung wieder beseitigt werden. Außerdem ist es denkbar, daß niedermolekulare migrationsfähige Kieselsäuren, welche die Aktivität der Katalysatoren erfahrungsgemäß beeinträchtigen, wieder in eine höhermolekulare, nicht migrationsfähige Form überführt werden.

Unter den genannten Metallen haben sich diejenigen der Gruppe IIA des Periodensystems, also die Erdalkalimetalle, darunter vor allem Calcium, als besonders wirksam erwiesen. Ebenfalls gut geeignet sind Aluminium und die Erdmetalle der Gruppe IIIB des Periodensystems einschließlich der Lanthaniden sowie die Metalle der Gruppe IVB des Periodensystems, also Titan, Zirkon und Hafnium. Aus wirtschaftlichen Gründen wird man im allgemeinen das Calcium bevorzugen.

Die Metalle können in den Salzen als Metallkationen wie $Cu^{++}$ oder $Ca^{++}$ oder als komplexe Kationen wie $(TiO_2)^{++}$ vorliegen.

Da die sauren Zentren des $Al_2O_3$, wie bereits dargelegt, von den Metallkationen besetzt werden und das $Al_2O_3$ demnach wie ein Ionenaustauscher wirkt, kommt es auf die Natur der Anionen grundsätzlich nicht an, da sie als Säuren oder Wasser in die wäßrige Lösung gehen. Da geringe Mengen der Anionen aber auch am $Al_2O_3$ adsorbiert bleiben und einige Anionen, z. B. die Halogenide, Sulfat und Phosphat das Verhalten des Katalysators beeinflussen, empfiehlt es sich, solche Verbindungen zu verwenden, deren Anionen bekanntermaßen wirkungsneutral sind, also z. B. Hydroxid, Carbonat, Nitrat oder die Anionen von Carbonsäuren wie Formiat oder Acetat. Da sich der Effekt der Vorbehandlung durch einen einfachen Test nachweisen läßt, wie aus den Ausführungsbeispielen hervorgeht, läßt sich auch der Einfluß eines bestimmten Anions unschwer durch einen Vorversuch ermitteln. Im allgemeinen wird man aus wirtschaftlichen Gründen Hydroxid als Anion bevorzugen oder, falls die Hydroxide zu schwerlöslich sind, Nitrat.

Eine geringe Löslichkeit reicht indes aus, weil es ohnehin zweckmäßig ist, die Behandlung des $Al_2O_3$ mit verdünnten wäßrigen Salzlösungen vorzunehmen. Bevorzugte Salzkonzentrationen liegen zwischen

2

0,01 und 1 Gew.%, besonders zwischen 0,1 und 0,6 Gew.%. Höhere Konzentrationen, etwa bis 10 Gew.%, sind jedoch ebenfalls möglich.

Im Zusammenhang mit der Vermutung, daß ein nicht erfindungsgemäß nachbehandeltes $Al_2O_3$ deshalb als Katalysatorträger ungeeignet ist, weil es saure Zentren aufweist, die ihrerseits auf die Silberrückgewinnung mittels Salpetersäure zurückzuführen sind, wurde beobachtet. daß die Konzentration der Behandlungslösung umso geringer sein kann, je geringer die Salpetersäurekonzentration war. Besonders bewährt hat sich die Ablösung des Silbers mit einer etwa 10-gew.%igen Salpetersäure, und dementsprechend erzielt man eine erfolgreiche Regenerierung des Trägers mit einer 0,1-0,6 gew.%igen Salzlösung.

Besonders bevorzugt wird eine bei Raumtemperatur gesättigte Calciumhydroxidlösung (ca. 0,12 Gew.% $Ca(OH)_2$ als Behandlungslösung.

Die Menge der Behandlungslösung ist unkritisch und beträgt aus praktischen Gründen etwa 1-5 l pro Liter (Schüttvolumen) des $Al_2O_3$.

Man nimmt die Behandlung zweckmäßigerweise bei Raumtemperatur vor und läßt die Lösung etwa 10-300 min auf das Trägermaterial einwirken. Danach trennt man die Lösung ab und trocknet das $Al_2O_3$ wie üblich, also etwa bei 120-300 °C.

Anstelle der Behandlung mit der Salzlösung gemäß Ausführungsform (a) des erfindungsgemäßen Verfahrens kann man das vom Silber befreite und gewaschene $Al_2O_3$-Trägermaterial gemäß Ausführungsform (b) auch für etwa 10-300 min auf 750-1 500 °C erhitzen, wobei die sauren Zentren vermutlich infolge von Wasserabspaltung zerstört werden und niedermolekulare Kieselsäuren in höhermolekulare übergehen.

Welche der beiden Ausführungsformen, die man selbstverständlich auch gemeinsam anwenden kann, zu bevorzugen ist, hängt von den betrieblichen Gegebenheiten ab. Methode (b) ist zwar einfacher, setzt aber das Vorhandensein hinreichend großer Hochtemperaturöfen voraus. Bei Methode (a) hingegen kommt man bei geringem Chemikalienverbrauch ohne besondere Apparaturen aus.

Beschichtet man den erfindungsgemäß regenerierten Träger anschließend nach einer der zahlreichen hierfür allgemein bekannten Methoden wieder mit Silber und gegebenenfalls mit hierfür üblichen weiteren Zusätzen, so erhält man Katalysatoren, die genauso wirksam sind wie solche, die unter Verwendung ungebrauchten Trägermaterials hergestellt worden sind.

## Beispiel

5 kg eines gebrauchten $Ag/Al_2O_3$-Katalysators, der 40 000 Betriebsstunden lang (etwa 5 Jahre) für die Herstellung von Ethylenoxid gedient hatte und keine wirtschaftliche Aktivität mehr aufwies, wurde in üblicher Weise bei Raumtemperatur mit 10-gew.%iger Salpetersäure vom Silber befreit, wonach das Trägermaterial dreimal mit vollentsalztem Wasser gewaschen wurde.

Je 100 g des Trägers wurden eine Stunde lang bei Raumtemperatur mit 500 g einer wäßrigen Metallsalzlösung der Konzentration k behandelt und anschließend 10 min lang bei 300 °C getrocknet.

Die so erhaltenen Trägermaterialien A'-N' wurden danach wie üblich mit einer aktiven Masse versehen, indem sie mit einer Lösung aus 11,5 g sec.-Butylamin, 3,5 g Wasser, 13,9 g Silbernitrat und 139 mg Lithiumnitrat imprägniert und anschließend im Umluftofen bei 220 °C getrocknet wurden. Diese « Grundkatalysatoren » wurden sodann mit einer Lösung aus 16,1 g Methanol, 400 mg sec.-Butylamin, 200 mg Hydrazinhydrat und 16,4 mg Caesiumhydroxid getränkt und abermals bei 200 °C im Umluftofen getrocknet.

Diese fertigen aktivierten Katalysatoren A-N enthielten neben den Bestandteilen des Trägers 8,0 Gew.% Silber, 0,015 Gew.% Lithium und 0,0015 Gew.% Caesium und entsprachen hinsichtlich ihrer Herstellung und ihrer Zusammensetzung dem ursprünglichen Kontakt, einem bewährten Katalysator des Standes der Technik.

Weitere Katalysatoren O-R wurden in analoger Weise, jedoch ohne die Vorbehandlung des Trägers mit einer Salzlösung hergestellt. Statt dessen wurde das nach der Salpetersäurebehandlung anfallende $Al_2O_3$ t min lang auf $\tau$ °C erhitzt.

Zum Vergleich wurden zwei weitere Katalysatoren X und Y hergestellt und zwar mit frischem $\alpha$-$Al_2O_3$ als Träger (X) und mit dem gebrauchten, aber keiner weiteren Behandlung unterzogenen Träger (Y).

Alle Katalysatoren A-R sowie X und Y wurden auf eine Teilchengröße von 0,5-0,6 mm Durchmesser zerkleinert und in üblicher Weise in einem Testreaktor mit einer Kontaktfüllung von 5 g auf ihre Wirksamkeit geprüft. Hierzu wurden pro Stunde bei 15 bar 15 l eines Gasgemisches aus 8 Vol.-% Sauerstoff, 30 Vol.-% Ethylen, 62 Vol.-% Stickstoff und 1 ppm Vinylchlorid durch den Kontakt geleitet. Die Temperatur wurde dabei so eingestellt, daß jeweils ein Sauerstoffumsatz von 50 % erzielt wurde. $T_1$ ist dabei die Temperatur nach 3-tägiger Versuchsdauer und $T_2$ die Temperatur nach 2-wöchiger Versuchsdauer. Die dazu gehörigen Selektivitäten $S_1$ und $S_2$ geben in Prozent an, wieviel des umgesetzten Ethylens zu Ethylenoxid reagiert hatten.

Alle Versuchsdaten sind der Tabelle zu entnehmen, welche die Gleichwertigkeit des erfindungsgemäß regenerierten $Al_2O_3$ mit frischem $Al_2O_3$ als Träger sowie die Überlegenheit des erfindungsgemäß regenerierten $Al_2O_3$ über nicht vorbehandeltes Material unter Beweis stellt.

3

Tabelle

| Vers. Nr. | Kat. | Salz | k Gew.% | °C | $T_1$ °C | $S_1$ % | $T_2$ °C | $S_2$ % |
|---|---|---|---|---|---|---|---|---|
| 1 | A | $Ca(OH)_2$ | 0,12 | – | 221 | 81,6 | 221 | 81,7 |
| 2 | B | $Ba(OH)_2$ | 0,75 | – | 223 | 81,1 | 223 | 81,1 |
| 3 | C | $Mg(Ac)_2$ | 0,50 | – | 220 | 81,4 | 220 | 81,6 |
| 4 | D | $Zn(Ac)_2$ | 0,50 | – | 221 | 80,9 | 221 | 80,8 |
| 5 | E | $Ca(NO_3)_3$ | 0,90 | – | 220 | 81,1 | 220 | 81,2 |
| 6 | F | $Mn(Ac)_2$ | 0,50 | – | 224 | 80,7 | 225 | 80,6 |
| 7 | G | $Al(NO_3)_3$ | 0,75 | – | 219 | 80,6 | 219 | 80,7 |
| 8 | H | $Pb(Ac)_2$ | 0,80 | – | 223 | 80,4 | 224 | 80,4 |
| 9 | I | $Ce(NO_3)_3$ | 0,90 | – | 219 | 81,2 | 219 | 81,1 |
| 10 | K | $Cu(Ac)_2$ | 0,50 | – | 232 | 80,0 | 233 | 80,2 |
| 11 | L | $(ZrO_2)(Ac)_2$ | 0,90 | – | 218 | 81,3 | 219 | 81,3 |
| 12 | M | $Ca(NO_3)_2$ | 0,75 | – | 220 | 81,4 | 220 | 81,5 |
| 13 | N | $Ca(HCO_3)_2$ | 0,60 | – | 219 | 81,7 | 219 | 81,6 |
| 14 | O | – | – | 750 | 220 | 81,2 | 223 | 81,0 |
| 15 | P | – | – | 1000 | 221 | 81,5 | 221 | 81,5 |
| 16 | Q | – | – | 1250 | 220 | 81,6 | 220 | 81,5 |
| 17 | R | – | – | 1500 | 219 | 81,4 | 219 | 81,6 |
| V1 | X | – | – | – | 220 | 81,5 | 220 | 81,5 |
| V2 | Y | – | – | – | 228 | 80,9 | 247 | 79,5 |

Ac = Acetat

**Patentanspruch**

Verfahren zur Regenerierung des $Al_2O_3$-Trägermaterials von $Ag/Al_2O_3$-Trägerkatalysatoren, welches nach Ablösung des Silbers von derartigen gebrauchten Katalysatoren mit Salpetersäure Ablösung des Silbers von derartigen gebrauchten Katalysatoren anfällt, dadurch gekennzeichnet, daß man das Trägermaterial

a) mit einer wäßrigen Lösung eines wasserlöslichen Salzes oder Hydroxides der Metalle der Gruppen IIA, IIIB oder IVB des Periodensystems oder des Aluminiums, Kupfers, Mangans, Zinks, Cadmiums, Zinns oder Bleis behandelt und das derart behandelte Material anschließend trocknet, und/oder daß man das Material

b) mindestens 10 min lang auf 750-1 500 °C erhitzt.

**Claim**

A process for the regeneration of the $Al_2O_3$ carrier material of $Ag/Al_2O_3$ supported catalysts which is obtained after removal of the silver from such spent catalysts with nitric acid, wherein the carrier material

a) is treated with an aqueous solution of a water-soluble salt or hydroxide of a metal of group IIA, IIIB or IVB of the Periodic Table or of aluminium, copper, manganese, zinc, cadmium, tin or lead, and the material treated in this manner is then dried, and/or the material

b) is heated at 750-1 500 °C for at least 10 minutes.

**Revendication**

4

**0 110 088**

Procédé pour la régénération du $Al_2O_3$ servant de matière support de catalyseurs à l'argent sur support d'$Al_2O_3$, tel qu'obtenu après la dissolution par l'acide nitrique de l'argent de catalyseurs usés de ce genre, caractérisé en ce que la matière support, soit

a) est traitée avec une solution aqueuse d'un hydroxyde ou d'un sel hydrosoluble des métaux des groupes IIA, IIIB et IVB du Système périodique ou de l'aluminium, du cuivre, du manganèse, du zinc, du cadmium, de l'étain ou du plomb, puis séchée, soit

b) est chauffée pendant au moins dix minutes entre 750 et 1 500 °C.